# EUROPEAN PATENT SPECIFICATION

(11) **EP 4 356 886 B1**
(45) Date of publication and mention of the grant of the patent: **11.03.2026**
(21) Application number: 23195020.5
(22) Date of filing: 04.09.2023
(51) Int. Cl.: A61F 13/15, B65H 43/00

(54) **PRODUCTION LINE OF ABSORBENT SANITARY ARTICLES**
PRODUKTIONSLINIE FÜR ABSORBIERENDE HYGIENEARTIKEL
LIGNE DE PRODUCTION D'ARTICLES SANITAIRES ABSORBANTS

(30) Priority: 18.10.2022 IT 202200021474
(43) Date of publication of application: 24.04.2024
(73) Proprietor: GDM S.p.A., 40133 Bologna (IT)
(72) Inventor: PIANTONI, Matteo, 40133 Bologna (BO) (IT); ZAVALLONI, Alessandro, 40133 Bologna (BO) (IT)
(74) Representative: Porta & Consulenti Associati S.p.A.

(56) References cited:
- WO-A1-2018/097171
- US-A1- 2013 304 246
- US-A1- 2019 258 233
- US-A1- 2019 282 408
- US-B2- 7 082 347

## Description

The invention concerns a production line of absorbent sanitary articles and a method for controlling the production of absorbent sanitary articles.

The absorbent sanitary articles are for example nappies for children, sanitary pads or tampons for women, pads for people suffering from incontinence and the like.

Typically, such articles are produced in a production line characterized by a high construction complexity, and at the same time the nappies themselves have a high structural complexity. For this reason, production can be affected by malfunctionings and defective articles.

EP 2 678 746 discloses a method of corrective action on the operation of a production line of absorbent sanitary articles in which it is provided to:
- detect at least one image of each article in output from the line;
- define by means of said image first parameters indicative of the positioning and/or of the assembly and/or of the shape of at least one respective component;
- detect a production defect in the event that at least one of said first parameters is outside a respective range of acceptability;
- identify second operating parameters of the line which are responsible for the definition of the first parameter resulted outside the respective range of acceptability;
- compare said operating parameters of the line with respective third reference parameters indicative of an optimal operation of the line;
- derive from the comparison a map of anomalous operating parameters;
- verify whether each combination of anomalous operating parameters is significant of a respective malfunctioning cause of the line, the respective cause falling within case studies of preset and defined malfunctioning causes;
- define, for each malfunctioning cause found, the corrective action to be taken to nullify the production defect.

The Applicant observes that the solution disclosed by EP 2 678 746 enables to detect the presence of a malfunctioning of the production line starting from a production defect detected by processing an image of an article in output from the line.

With respect to such a solution, the Applicant has perceived the need to provide an alternative method for controlling the production of absorbent sanitary articles.

In particular, the Applicant has perceived the need to improve the control of the production of absorbent sanitary articles, in particular in terms of reliability, precision and quality.

The Applicant has found that the aforesaid need can be met by an automated production control based on a system for monitoring current values of operating parameters which are indicative of the operation of production devices of the line and on a processing of the current values of the monitored operating parameters which is adapted to detect any anomalous operating parameters and to derive therefrom a current malfunctioning cause of the line, among a plurality of possible malfunctioning causes.

The present invention therefore concerns, in a first aspect thereof, a production line of absorbent sanitary articles.

The production line comprises production devices configured to perform respective production operations on the absorbent sanitary articles being processed along the production line.

The production line comprises sensors adapted to detect current values of operating parameters which are indicative of the current operation of at least one of the production devices.

The production line comprises a control unit comprising a memory and a processor configured to perform a comparison between the current values of the operating parameters detected by the sensors and respective predetermined reference values stored in said memory. In the presence, among said operating parameters, of at least one anomalous operating parameter whose current value does not comply with the respective predetermined reference value, said processor is preferably configured to identify, among a plurality of possible malfunctioning causes of the line, at least one current malfunctioning cause responsible for said at least one anomalous operating parameter. Preferably, the processor is configured to perform said comparison independently of a detection of any defects performed on the absorbent sanitary articles.

Preferably, the processor is configured to identify said at least one current malfunctioning cause starting from said at least one anomalous operating parameter by means of decision algorithms which are based on at least one of the following information stored in said memory: known and/or self-learned data related to correlations between said at least one anomalous operating parameter and said plurality of possible malfunctioning causes of the line; current and/or historical information manually provided by an operator in association with said at least one anomalous operating parameter and/or said possible malfunctioning causes of the line; known and/or self-learned logic rules.

The present invention concerns, in a second aspect thereof, a method for controlling the production of absorbent sanitary articles in a production line.

It is provided to detect current values of operating parameters which are indicative of the current operation of at least one production device adapted to perform respective production operations on the absorbent sanitary articles being processed along the production line.

It is provided to perform a comparison between the current values of the detected operating parameters and respective predetermined reference values.

In the presence, among said operating parameters, of at least one anomalous operating parameter whose current value does not comply with the respective predetermined reference value, it is provided to identify, among a plurality of possible malfunctioning causes of the line, at least one current malfunctioning cause responsible for said at least one anomalous operating parameter.

Said comparison is performed independently of a detection of any defects performed on the absorbent sanitary articles.

Said at least one current malfunctioning cause is identified starting from said at least one anomalous operating parameter by means of decision algorithms which are based on at least one of the following information: known and/or self-learned data related to correlations between said at least one anomalous operating parameter and said plurality of possible malfunctioning causes of the line; current and/or historical information manually provided by an operator in association with said at least one anomalous operating parameter and/or with said plurality of possible malfunctioning causes of the line; known and/or self-learned logic rules.

The Applicant observes that, according to the invention, the current malfunctioning cause of the line is identified based on parameters that are directly connected to the operation of the production devices, independently of a detection of defects performed on the absorbent sanitary articles.

This advantageously allows to identify the malfunctioning causes of the line in a reliable and precise way, considering that there are malfunctionings of the line that do not affect the quality of the articles produced or that are identifiable with difficulty starting from a visual inspection of the articles produced by the line. These are, for example, malfunctionings (such as wear or loss of energy efficiency of a machine) that do not necessarily generate defects on the absorbent sanitary articles produced by the line; or there are malfunctionings that generate defects (see, for example, defects related to the tightness of a seal or to the degree of absorbency of the articles produced) which - being not visible - are hardly detectable by traditional quality control systems of the *machine vision* type. The latter defects, in order to be identified, would require advanced and expensive inspection devices or controls performed in the laboratory, outside the production line, on sample products.

The Applicant further observes that, by providing a control method that operates upstream of any detection of defects, the invention advantageously allows to have real-time information on malfunctioning causes that could generate defects in the articles and, therefore, promptly signal the presence of possible defective articles and/or intervene immediately on the production line in order to prevent the production of defective articles.

In addition, the Applicant observes that given the high constructional complexity of the production line of absorbent sanitary articles and the high structural complexity of the same articles, an anomalous operating parameter could be generated by different possible malfunctioning causes of the line. Tracing back, among a plurality of possible malfunctioning causes, to the malfunctioning cause that is currently responsible for the anomalous operating parameter can be a non-immediate operation and require the intervention of experienced operators, provided with a high knowledge of the operation of the line, of the most frequent problems that can be found in the production lines of absorbent sanitary articles and with a good historical memory of malfunctioning causes identified in the past in association with predetermined values of anomalous operating parameters. Consider, for example, the case of identifying an anomalous operating parameter related to an irregular vibration of a device. This anomaly could be generated by different malfunctioning causes of the line such as, for example, the wear or breakage of a piece of the device itself, the malfunctioning of upstream devices that have caused the presence in the article of a seal or of a material with a thickness greater or smaller than expected, the blockage or the misalignment of a piece of the device itself and the like.

According to the invention, all this is overcome thanks to the decision algorithms that allow - without the intervention of experienced operators - to automatically identify, among a plurality of possible malfunctioning causes, the one that has currently caused the anomalous operating parameter.

Overall, the objectives set forth above of improving the control of the production of absorbent sanitary articles, in particular in terms of reliability, precision and quality are achieved.

The present invention further concerns, in a further aspect thereof, a method of predictive detection of any defects in absorbent sanitary articles being processed along a production line and/or in finished absorbent sanitary articles in output from the production line.

Preferably, it is provided to detect current values of operating parameters which are indicative of the current operation of at least one production device adapted to perform respective production operations on the absorbent sanitary articles being processed in the production line.

Preferably, it is provided to perform a comparison between the current values of the detected operating parameters and respective predetermined reference values.

Preferably, in the presence, among said operating parameters, of at least one anomalous operating parameter whose current value does not comply with the respective predetermined reference value, it is provided to identify, among a plurality of possible malfunctioning causes of the line, at least one current malfunctioning cause responsible for said at least one anomalous operating parameter, said at least one current malfunctioning cause being identified starting from said at least one anomalous operating parameter by means of decision algorithms which are based on at least one of the following information: known and/or self-learned data related to correlations between said at least one anomalous operating parameter and said plurality of possible malfunctioning causes of the line; current and/or historical information manually provided by an operator in association with said at least one anomalous operating parameter and/or with said plurality of possible malfunctioning causes of the line; known and/or self-learned logic rules.

Preferably, by means of said decision algorithms, it is provided to make a prediction of any defects starting from said at least one current malfunctioning cause identified and based on at least one of the following information: known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

By *"*absorbent sanitary article being processed" or "article being processed" is meant a semi-finished absorbent sanitary article at any stage of the relative production process within a production line, including individual materials and individual elements forming the absorbent sanitary article.

By the term *"*finished absorbent sanitary article" or "finished article" is meant a finished absorbent sanitary article obtained at the end of the production process.

By *"*absorbent sanitary article" or *"*article*"* is meant a finished absorbent sanitary article or an absorbent sanitary article being processed.

By *"*malfunctioning*"* referred to a production line is meant a malfunctioning that may concern any operating device in the production line, including both a production device that is configured to perform a respective production operation on the absorbent sanitary articles being processed along the production line and an accessory device with respect to production, such as a device adapted to maintain adequate boundary conditions within the plant in which the production line is housed, such as for example a device adapted to maintain a certain level of temperature and/or humidity within the plant.

Such malfunctioning could generate a defect in the absorbent sanitary articles being processed along a production line and/or in finished absorbent sanitary articles in output from the production line and/or cause a stop (preferably automatic) of the line or of the malfunctioning production device.

By *"*defect*"* is meant to indicate an imperfection that is present or may occur in an absorbent sanitary article with respect to a predefined standard.

By *"*advancement direction" referred to an article within a production line is meant to indicate a direction of movement of that article within that line. For example, in the case of an article moved by transport members (comprising, for example, a conveyor belt) it is meant to indicate a direction of movement of said transport members (in the example of said conveyor belt).

The present invention may have, in the aspects discussed above, at least one of the preferred characteristics disclosed below. These characteristics may therefore be present individually or in combination with each other, unless expressly stated otherwise.

Preferably, the method of predictive detection is a computer-implemented method.

Preferably, the production control method is a computer-implemented method.

Preferably, the processor is configured to perform said comparison between the current values of the detected operating parameters and the respective reference values continuously.

Preferably, the processor is configured to perform said comparison between the current values of the detected operating parameters and the respective reference values independently of a detection of any defects performed directly on the absorbent sanitary articles being processed along the line and on the finished absorbent sanitary articles, in output from the line.

Preferably, the processor is configured to perform said comparison between the current values of the detected operating parameters and the respective reference values independently of a detection of any defects performed by means of inspection devices adapted to directly inspect the absorbent sanitary articles being processed along the line and on the finished absorbent sanitary articles, in output from the line.

The predetermined reference values can be represented by individual values (in this case the comparison is performed to verify that the current values of the operating parameters detected by the sensors are greater or lower than the respective reference values) or by ranges of values (in this case the comparison is performed to verify that the current values of the operating parameters detected by the sensors are inside or outside the respective ranges of reference values).

In one embodiment, one or more of the sensors are adapted to detect current values of operating parameters whose anomaly with respect to the respective predetermined reference values generates a stop (preferably automatic) of the line or of the relative production device.

In a preferred embodiment, the line comprises at least one inspection device configured to acquire article data directly correlated to the absorbent sanitary articles being processed along the production line and/or to the finished absorbent sanitary articles, in output from the line.

The article data can relate, for example, to the appearance, shape, weight, positioning, dimensions, contours of the articles being processed along the production line and/or to the finished absorbent sanitary articles, in output from the line.

Said at least one inspection device may, for example, be selected from: an image acquisition device, a weight sensor, a proximity sensor, a photoelectric sensor, a contour sensor, a displacement sensor, a position sensor and a quantity sensor.

Preferably, the processor is configured to perform said detection of any defects directly on the absorbent sanitary articles being processed along the production line and/or on the finished absorbent sanitary articles in output from the line, by processing the article data acquired by said at least one inspection device.

Preferably, the processor is configured to perform (i.e. trigger) said comparison independently of said detection of any defects performed by processing said article data. That is, the processor is configured to perform said comparison continuously regardless of whether or not a defect is detected by processing said article data. In particular, the detection of a defect by processing said article data is not necessary for the purposes of the activation of said comparison. However, this does not exclude that data related to defects detected by processing of said article data may be used by said decision algorithms.

In a preferred embodiment, the line comprises an image acquisition device (e.g., a camera) configured to acquire images of the absorbent sanitary articles being processed along the production line and/or images of the finished absorbent sanitary articles, in output from the line.

Preferably, the processor is configured to perform said detection of any defects directly on the absorbent sanitary articles being processed along the production line and/or on the finished absorbent sanitary articles in output from the line, by processing the images acquired by the image acquisition device.

Preferably, the processor is configured to perform (i.e. trigger) said comparison independently of said detection of any defects performed by processing said images. That is, the processor is configured to perform said comparison continuously regardless of whether or not a defect is detected by processing said images. In particular, the detection of a defect by processing said images is not necessary for the purpose of the activation of said comparison. However, this does not exclude that data related to defects detected by processing said images may be used by said decision algorithms.

Preferably, the processor is configured to store in said memory said at least one current malfunctioning cause identified in association with said at least one anomalous operating parameter so as to enrich the self-learned data related to correlations between said at least one anomalous operating parameter and said plurality of possible malfunctioning causes of the line.

Preferably, the processor is configured to perform, by means of said decision algorithms, a prediction of any defects in the absorbent sanitary articles starting from said at least one current malfunctioning cause identified and based on at least one of the following information stored in said memory: known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

This prediction of any defects is distinct from the detection of defects performed by processing the article data acquired by said at least one inspection device or, specifically, by processing the images acquired by the image acquisition device and can be performed in addition or alternatively to it.

Preferably the prediction concerns both any defects that might occur (but are not yet present) in the absorbent sanitary articles being processed along the production line and/or in the finished absorbent sanitary articles in output from the line and defects that are already present in such articles.

Preferably, the processor is configured to store in said memory any predicted production defects so as to enrich the self-learned data related to said correlations between said at least one current malfunctioning cause identified and said at least one known possible defect.

Preferably, the processor is configured to define, by means of said decision algorithms, a corrective action to be taken to remedy said at least one current malfunctioning cause identified.

The corrective action to be taken may concern one or more of the production devices or one or more of accessory devices (with respect to production) associated with the production line, which are involved in the current malfunctioning cause identified.

For example, an accessory device could be a device adapted to maintain adequate boundary conditions within the plant in which the production line is housed, such as for example a device adapted to maintain a certain level of temperature and/or humidity within the plant.

Preferably, the line comprises actuators adapted to implement the corrective action to be taken.

Preferably, the processor is configured to automatically convert the corrective action to be taken into a control signal for the actuators.

Preferably the actuators are operatively associated with said control unit.

Preferably, the actuators are operatively associated with at least one of the production devices.

Preferably, the actuators are configured to intervene on the operation of at least one of the production devices based on the control signal coming from the processor.

Preferably, the actuators are operatively associated with one or more accessory devices with respect to the production of the articles, which are present within the production line.

Preferably, the actuators are configured to intervene on the operation of one or more of the accessory devices based on the control signal coming the processor.

Preferably, the production devices comprise at least one of:
- transport members adapted to support and move the absorbent sanitary articles being processed along the line;
- feeding devices adapted to feed elements of the absorbent sanitary articles being processed (for example in the form of tapes) and to place them, at least partially mutually overlapping, on a supporting surface of the transport members;
- retaining members configured to retain in position the elements placed on the supporting surface of the transport members, preferably comprising suction devices active on retaining holes formed on the supporting surface of the transport members;
- at least one fixing device adapted to fix the elements of the absorbent sanitary articles being processed together (for example by sealing and/or gluing);
- at least one cutting device adapted to cut elements of the absorbent sanitary articles being processed and/or a continuous strip of the absorbent sanitary articles being processed joined together, resulting from the production process, into individual articles.

Preferably, the sensors comprise at least one of: temperature sensor; pressure sensor; vibration sensor (e.g., accelerometer); position sensor; weight sensor; quantity sensor; flow sensor (e.g., flow meter); vacuum level sensor; air jet status sensor; optical sensor; force sensor (e.g., load cells); speed sensor; torque sensor; current sensor.

Preferably, the operating parameters detected by the plurality of sensors comprise at least one of: temperature; pressure; vibration; position of objects; weight; quantity; vacuum level; status of air jets; impact energy level of the cutting device; level of cleanliness; presence or quantity of material at a predetermined position; feeding tension of the elements of the absorbent sanitary articles being processed; speed; torque (e.g. of a motor) and current absorbed (e.g. by a motor).

Preferably, said decision algorithms are implemented by means of artificial intelligence, expert system or preset and defined logics and correlations.

Preferably, said decision algorithms are assisted by suitable machine learning and/or statistical algorithms.

Preferably, in the case of several anomalous operating parameters, the processor is configured to identify said at least one current malfunctioning cause both starting from each individual anomalous operating parameter and starting from various possible combinations of said anomalous operating parameters.

Preferably, in the case of identifying several malfunctioning causes, the processor is configured to make the prediction of any defects both starting from each individual malfunctioning cause identified and starting from various possible combinations of malfunctioning causes identified.

In a preferred embodiment, it is provided to acquire article data directly correlated to the absorbent sanitary articles being processed along the production line and/or to the finished absorbent sanitary articles, in output from the line.

The article data can relate, for example, to the appearance, shape, weight, positioning, dimensions, contours of the articles being processed along the production line and/or to the finished absorbent sanitary articles, in output from the line.

Preferably, it is provided to detect any defects on the absorbent sanitary articles being processed along the production line and/or on the finished absorbent sanitary articles, in output from the line, by processing said article data.

Preferably, it is provided to perform said comparison continuously (for example according to a predefined cadence) independently of said detection of any defects performed by processing said article data.

In a preferred embodiment, it is provided to acquire images of the absorbent sanitary articles being processed along the production line and/or images of the finished absorbent sanitary articles, in output from the line.

Preferably, it is provided to detect any defects on the absorbent sanitary articles being processed along the production line and/or on the finished absorbent sanitary articles, in output from the line, by processing said images.

Preferably, it is provided to perform said comparison continuously (for example according to a predefined cadence).

Preferably, it is provided to perform said comparison continuously (for example according to a predefined cadence) independently of said detection of any defects performed by processing said images.

Preferably, it is provided to store in said memory said at least one current malfunctioning cause identified in association with said at least one anomalous operating parameter so as to enrich the self-learned data related to correlations between said at least one anomalous operating parameter and said plurality of possible malfunctioning causes of the line.

Preferably, it is provided to perform, by means of said decision algorithms, a prediction of any defects that could occur or be already present in the absorbent sanitary articles starting from said at least one current malfunctioning cause identified and based on at least one of the following information stored in said memory: known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

Preferably, it is provided to store in said memory any predicted production defects so as to enrich the self-learned data related to said correlations between said at least one current malfunctioning cause identified and said at least one known possible defect.

Preferably, any defects predicted by means of said decision algorithms relate to at least one of: tightness of a seal, tightness of a gluing, degree of absorbency and distribution or quantity of material.

The defectiveness or not of a tightness of a seal or of a gluing can relate to the maximum stress (such as, for example, tensile force) that the seal is able to withstand until breakage.

Preferably, it is provided to define, by means of said decision algorithms, a corrective action to be taken to remedy said at least one current malfunctioning cause identified.

Generally, the detected current malfunctioning cause concerns one or more of the production devices or other accessory device (with respect to production) associated with the production line, which is different from the production device to which the anomalous operating parameter identified is associated.

The corrective action to be taken may concern one or more of the production devices and/or other accessory device (with respect to production) associated with the production line, which are involved in the current malfunctioning cause identified.

Preferably, it is provided to implement the defined corrective action by intervening on one or more of the production devices or other accessory device.

Preferably, the article comprises a plurality of elements.

Preferably, said elements are at least partly strip-shaped.

The elements can be distinguishable into base elements and additional elements.

The base elements preferably comprise a first sheet of permeable material and a second sheet of impermeable material, which are intended to define, respectively, the inner face and the outer face of the article.

The first sheet and the second sheet are typically mutually overlapping and between them there is interposed a third main element constituted by an absorbent padding.

The additional elements, which may vary in number and shape, preferably comprise front and/or rear flaps, acquisition and distribution layers or reinforcement layers or other discrete elements typically envisaged in the absorbent sanitary articles between the first sheet and the second sheet.

Preferably, within the production line, the absorbent sanitary articles being processed can be moved along an advancement direction.

Preferably, the transport members are adapted to support and move the absorbent sanitary articles being processed along said advancement direction.

In one embodiment, the transport members comprise at least one conveyor belt.

Preferably, the conveyor belt can be moved along the advancement direction for the movement of the articles being processed within the production line.

The conveyor belt may have an at least partly flat and/or curved supporting surface.

The advancement direction may be at least partly straight and/or curved.

Further features and advantages of the present invention will become clearer from the following detailed description of a preferred embodiment thereof, made with reference to the appended drawings and provided by way of indicative and non-limiting example, in which:
- Figure 1 schematically shows a production line of absorbent sanitary articles according to an embodiment of the invention;
- Figure 2 represents, in plan, a schematic example of an absorbent sanitary article that can be made by the line of Figure 1.

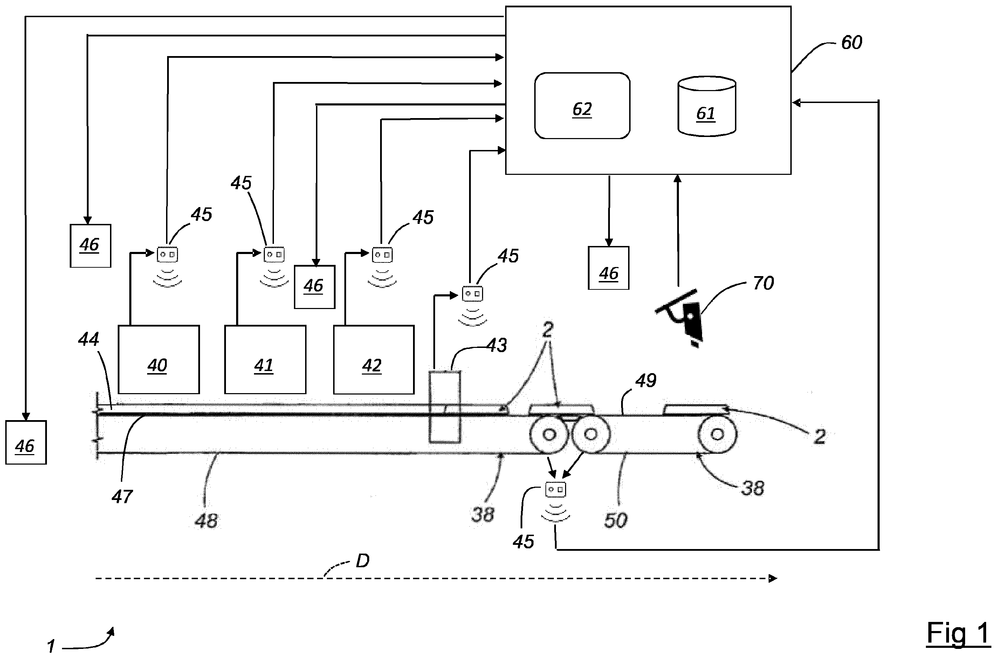

Figure 1 schematically shows a production line 1 of absorbent sanitary articles 2 according to an embodiment of the invention.

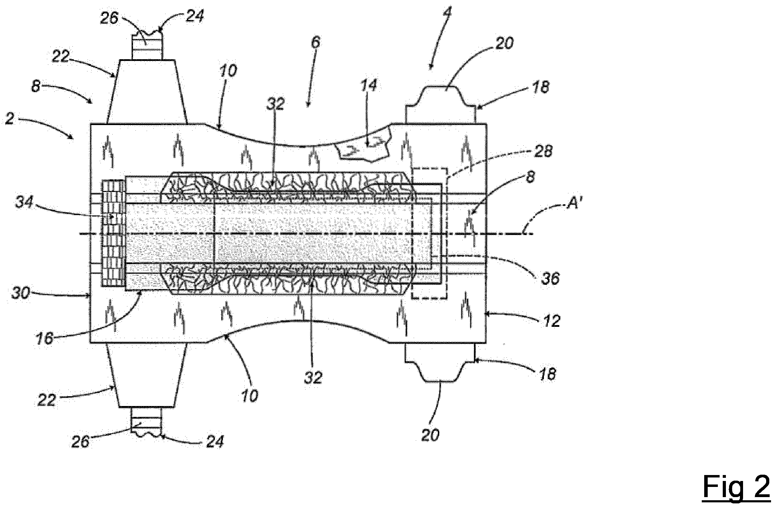

With reference to Figure 2, the absorbent sanitary articles 2 have a substantially rectangular shape. In the specific example the articles 2 are nappies for children, however, in the present invention and according to variants immediately deducible from the context and from what is disclosed below, the articles 2 may be sanitary pads for women, pads for senile incontinence or the like.

The articles 2 comprise, in alignment according to a longitudinal axis thereof, indicated with A', a front portion 4, wearable on the front part of the body of the user, a rear portion 8, wearable on the rear part of the body of the user, and a central portion 6, arranged between the front part and the rear part and wearable between the legs of the user. At the central portion 6 there is provided a recess 10, or leg line, defined by two arcuate sections that are symmetrical with respect to the axis A*'*.

The absorbent sanitary articles 2 are formed by a plurality of elements, distinguishable into base elements and additional elements. In particular, the base elements are assembled together in such a way as to define a base article 2, whose elements are arranged according to a preset and defined scheme. Similarly, the additional elements are applied on the base article during and/or after the definition thereof, according to a preset and defined scheme, so as to realize a finished article 2.

The base elements are constituted by a first sheet 12 of permeable material (non-woven fabric) and a second sheet 14 of impermeable material, which are intended to define, respectively, the inner face and the outer face of the article 2.

The sheets 12 and 14 are mutually overlapping and between them there is interposed a third main element constituted by an absorbent padding 16. The padding 16 is formed by cellulose fibre and superabsorbent polymer (SAP) granules dispersed therein.

The additional elements, which may vary in number and shape, are disclosed below with reference to the absorbent sanitary article 2 illustrated in Figure 2.

18 indicates two first shaped wings, each having a respective lobe 20, fixed to the inner face of the second impermeable sheet 14 and extending transversely to the axis A' from the front portion 4 of the article 2, and 22 indicates two shaped closing wings, parallel to the first wings 18, extending from the rear portion 8 of the article 2.

To each of the second closing wings 22 there is applied a third flap 24 provided with an adhesive strip 26, with a development parallel to the axis A', intended to adhere, in use, to a corresponding front strip 28 applied to the front portion 4 of the outer face of the second sheet 14. The wings 22 equipped with the flaps 24 constitute, together with the front strip 28, means for closing the article 2.

The adhesive strip 26 can be replaced, according to a variant, with a Velcro^{®} strip.

To the first sheet 12 of permeable material there are sealed laterally two strips 30 of impermeable material for thickening and expanding the longitudinal edges thereof. The strips 30 are provided at their intermediate section with an elasticized portion 32.

A further additional component is constituted by an elastic band 34 applied, transversely to the axis A*'*, to the inner face of the second sheet 14 at the rear portion 8 of the article 2.

At the inner face of the first sheet 12 of permeable material, in contact with the absorbent padding 16 and sealed along the contour of the latter, there is provided a sheet 36 of absorbent material, defined as "acquisition layer", having the function of making the absorbent power of the surface of the absorbent padding 16 uniform.

With reference to Figure 1, the production line 1 comprises a plurality of production devices 38, 40, 41, 42, 43 of known type adapted to perform respective production operations on the absorbent sanitary articles 2 being processed along the line 1.

Such operations may comprise, for example, support and movement; feeding of material constituting the base elements and the additional elements and/or the elements themselves; cutting the materials to make the individual elements or to shape the elements already applied; placing the base elements and the additional elements in partial or total mutual overlapping; fixing the various base and additional elements by gluing and/or sealing; cutting a continuous strip 44 of the absorbent sanitary articles 2 being processed joined together, resulting from the production process, into individual articles, and the like.

In the embodiment illustrated in Figure 1, the production devices comprise transport members 38 adapted to support and move, along an advancement direction D, the absorbent sanitary articles 2 being processed along the line 1.

In the illustrated embodiment, the advancement direction D is straight.

The transport members 38 may comprise a plurality of conveyor belts. In particular, in Figure 1 there are indicated a first conveyor belt 48 with a respective supporting surface 47 and a second conveyor belt 50 with a respective supporting surface 49.

In the illustrated embodiment, the production devices also comprise feeding devices (indicatively and schematically illustrated in the figure with a single block 40), adapted to feed material constituting the base elements and the additional elements of the articles 2 being processed and/or the elements themselves and to place them, at least partially mutually overlapping, on a supporting surface 47 of the transport members 38.

For example, in the embodiment illustrated in Figure 1, the supporting surfaces 47 and 49 are flat.

In the embodiment illustrated in Figure 1, the production devices also comprise retaining members (indicatively and schematically illustrated in the figure with a single block 41) configured to retain in position the elements/materials placed on the supporting surface 47 of the first conveyor belt 48 and to retain in position the finished articles 2 on the transport surface 49 of the second conveyor belt 50. For example, the retaining members 41 comprise suction devices (not illustrated) active on retaining holes (not illustrated) formed on at least part of the supporting surface 47 of the first conveyor belt 48 and on at least part of the transport surface 49 of the second conveyor belt 50.

In the embodiment illustrated in Figure 1, the production devices also comprise at least one fixing device (indicatively and schematically illustrated in the figure with a single block 42) adapted to fix the base and additional elements together by gluing and/or sealing.

Said at least one fixing device 42 may comprise an ultrasonic sealing device.

In an alternative embodiment, said at least one fixing device 42 may comprise a thermomechanical sealing device.

In addition or alternatively, said at least one fixing device 42 may comprise a gluing device comprising a glue dispensing device associated with a tank (not illustrated) for the glue. In one embodiment, said glue dispensing device may comprise inside it a small secondary glue tank adapted to draw the glue, by means of a suitable drawing mechanism, from an external primary glue tank.

In the embodiment illustrated in Figure 1, the production devices also comprise at least one cutting device (indicatively and schematically illustrated in the figure with a single block 43) adapted to perform the aforesaid cutting operations. In particular, Figure 1 depicts a device adapted to make cuts or transverse weakening lines (with respect to the advancement direction D) of the continuous strip 44 of absorbent sanitary articles being processed 2 in such a way as to be able to subsequently separate or make the absorbent sanitary articles 2 easily separable.

One or more of the production devices 40, 41, 42, 43 may be made by an individual device or by separate devices. Such an individual device or such separate devices may comprise one or more forming drums (not illustrated) which are rotatable about respective axes of rotation. In such a case, at least one between the first conveyor belt 48 and the second conveyor belt 50 may be wholly or partly replaced by transport surfaces of such forming drums.

The supporting surfaces 47 and 49 of the transport members 38 may thus be at least partly flat and/or curved. Furthermore the advancement direction D may be at least partly straight and/or curved.

The production line 1 also comprises sensors 45 distributed along the line 1. The sensors 45 are operatively associated with the production devices 40, 41, 42, 43, 38. In particular, the sensors 45 are configured to detect current values of operating parameters of the respective production devices 40, 41, 42, 43, 38.

The operating parameters of the respective production devices 40, 41, 42, 43, 38 are linked to the process of positioning, assembling and/or shaping the various elements constituting the article 2.

For example, the sensors 45 may comprise one or more temperature sensors, for example for measuring the temperature of the glue contained in the tank (not illustrated) associated with said at least one fixing device 42; one or more pressure sensors, for example adapted to measure a vacuum level created by the suction devices of the retaining members 41 and/or the pressure of the glue dispensed by said at least one fixing device 42; one or more vibration sensors such as, for example, an accelerometer adapted to measure, for example, the vibrations of a piece (such as, for example, the supporting surface 47 of the first conveyor belt 48) of a production device 38, 40, 41, 42, 43; one or more position sensors, for example adapted to measure the position of an object with respect to a reference or the mutual position of objects at one or more of the production devices 38, 40, 41, 42, 43. For example, the position sensors may be adapted to measure the position of a roller (not shown) of the feeding devices 40 with respect to an application point and/or the opening/closing of a shutter of the suction devices of the retaining members 41. The sensors 45 may further comprise one or more weight sensors (e.g. load cells), for example adapted to control the weight of the glue contained in the tank associated with said at least one fixing device 42 or the presence/absence/quantity of material at a predetermined position; one or more flow sensors such as, for example, a flow meter, for example adapted to measure the quantity of glue dispensed by said at least one fixing device 42; one or more air jet status sensors, for example adapted to detect the status (active/inactive/intensity) of the air jets created by the suction devices of the retaining members 41 (for example by measuring the pressure of the air upstream by the suction devices of the retaining members 41); one or more optical sensors, for example adapted to detect the level of cleanliness at a production device 38, 40, 41, 42, 43; one or more force sensors (for example load cells), for example adapted to detect the feeding tension of the elements/materials of the absorbent sanitary articles fed by the feeding devices 40 and/or to verify whether compression members/rollers at the feeding devices 40 are operating correctly. Load cells can, for example, be used also/or to detect the impact energy level of said at least one cutting device 43. In addition or alternatively, the sensors 45 may comprise one or more sensors adapted to detect operating parameters of one or more motors present at least at one production device 38, 40, 41, 42, 43, such as the torque, the temperature and the absorbed current.

In a preferred embodiment illustrated in Figure 1, the production line 1 also comprises an image acquisition device 70 (e.g., a camera) configured to acquire images of the finished absorbent sanitary articles 2, in output from the line 1.

The image acquisition device 70 may be arranged facing the second conveyor belt 50, as illustrated in Figure 1, to detect at least one image of each finished article 2, i.e. already separated from the continuous strip 44 of articles 2 joined together, or, according to a variant not illustrated, the image acquisition device 70 may be arranged facing the first conveyor belt 48 to detect at least one image of each article 2 when it must still be separated from the continuous strip 44 of articles 2 joined together. In both cases, the image acquisition device 70 is arranged at the output of the line 1 to detect at least one image of each article 2 already defined relatively to the composition and assembly of the base and additional elements thereof.

The production line 1 also comprises a control unit 60 comprising a memory 61 and a processor 62.

The processor 62 is configured to implement a control method according to an embodiment of the present invention.

The control unit is operatively connected (by means of a suitable wired and/or wireless connection) to the sensors 45 and to the image acquisition device 70.

In the preferred embodiment illustrated in Figure 1, the processor 62 is configured to detect any defects present in the finished absorbent sanitary articles 2, in output from the line. The detection of defects is performed by processing the images acquired by the image acquisition device 70 by means of suitable *machine vision* algorithms known in the art.

Typical defects that can be detected by said image processing may comprise: wrong alignment of the absorbent padding 16, wrong orientation of the front strip 28, wrong positioning of the closing wings 22 and wrong execution of the cut of the recess 10. These defects generally concern the positioning, the assembly and/or the shape of the various elements constituting the article 2 and are directly visible in the images acquired by the image acquisition device 70 and directly obtainable therefrom.

The processor 62 is further configured to perform continuously a comparison between the current values of the operating parameters detected by the sensors 45 and respective predetermined reference values stored in the memory 61.

When at least one of the detected current values does not comply with the respective predetermined reference value, the processor 62 labels the respective operating parameter as an anomalous operating parameter and is configured to identify, among a plurality of possible malfunctioning causes of the line 1, at least one current malfunctioning cause that is responsible for the anomalous operating parameter.

The malfunctioning cause may concern any operating device in the production line, including both a production device 38, 40, 41, 42, 43 and an accessory device (not illustrated) with respect to production, such as a device adapted to maintain adequate boundary conditions within the plant in which the production line 1 is housed, such as for example a device adapted to maintain a certain level of temperature and/or humidity within the plant.

The current malfunctioning cause identified concerns one or more of the production devices 38, 40, 41, 42, 43 and/or one or more of the accessory devices, which may be different from the production device 38, 40, 41, 42, 43 for which the anomalous operating parameter was detected.

In general, the anomalous operating parameter is not directly correlated to the current malfunctioning cause, which can be identified among a plurality of possible malfunctioning causes that could have generated such anomalous operating parameter.

The processor 62 is configured to identify said at least one current malfunctioning cause starting from said anomalous operating parameter by means of decision algorithms which are based on at least one of the following information stored in said memory: known and/or self-learned data related to correlations between said at least one anomalous operating parameter and said plurality of possible malfunctioning causes of the line; current and/or historical information manually provided by an operator in association with said at least one anomalous operating parameter and/or said possible malfunctioning causes of the line; known and/or self-learned logic rules.

According to the invention, the comparison between the current values of the operating parameters detected by the sensors 45 and respective reference values is not activated by the detection of defects performed by processing the images acquired by the image acquisition device 70. The processor is in fact configured to perform the comparison continuously, independently of said detection, that is to say regardless of whether or not a defect is detected by processing said images. However, this does not exclude that data related to defects detected by processing said images may be used by said decision algorithms.

The aforesaid decision algorithms can be implemented by means of techniques known in the art that make use of artificial intelligence, expert system or preset and defined logics as well as a modelling based on a study of the most frequent defects and problems that can be found in the production lines of absorbent sanitary articles.

In addition, the decision algorithms can be assisted by suitable statistical algorithms and suitable machine learning algorithms.

Preferably, in the case of several anomalous operating parameters, the processor 62 is configured to identify the current malfunctioning cause starting from both each individual anomalous operating parameter and from various possible combinations of said anomalous operating parameters.

Preferably, the processor 62 is configured to store in the memory 61 each current malfunctioning cause identified in association with the respective anomalous operating parameter(s) so as to enrich the self-learned data related to correlations between anomalous operating parameters and possible malfunctioning causes of the line.

In a preferred embodiment, the processor 62 is further configured to make, by means of the aforesaid decision algorithms, a prediction of any defects that might occur or be already present in the absorbent sanitary articles being processed along the line 1 and/or in the finished absorbent sanitary articles in output from the line 1. Such prediction is performed starting from said at least one current malfunctioning cause identified and based on at least one of the following information stored in said memory: known and/or self-learned data related to correlations between said at least one current malfunctioning cause identified and at least one possible defect; current and/or historical information manually provided by an operator in association with said at least one current malfunctioning cause identified and/or said at least one possible defect; known and/or self-learned logic rules.

For example, this prediction can be performed with the aid of a database in which every possible malfunctioning cause is correlated to a related defect. This database can be preset and defined on the basis of a modelling based on a study of known and/or self-learned data related to the most frequent defects and problems that can be found in the production lines of absorbent sanitary articles.

The processor 62 is also configured to store in the memory 61 any production defects thus predicted so as to enrich the self-learned data related to said correlations between malfunctioning causes and possible production defects.

The Applicant observes that this preferred embodiment advantageously allows to predict, in line and in an automated way, also defects that - not being detectable starting from a visual inspection of the articles - are hardly detectable starting from a processing of the images acquired by the image acquisition device 70.

This embodiment thus allows, alternatively or preferably in addition to the detection of defects performed starting from a processing of the images acquired by the image acquisition device 70, to perform a reliable and precise control of the production of articles 2.

Examples of non-visible defects that can be predicted by means of said decision algorithms can relate to: tightness of a seal, tightness of a gluing, degree of absorbency of the absorbent padding 16 and distribution or quantity of material in the absorbent padding 16.

Once said at least one current malfunctioning cause has been identified, the processor 62 is configured to perform at least one of the following actions: generate an alarm signal; generate a warning signal that the line 1 is making potentially defective articles; generate a warning signal on the need to discard the articles currently being produced; automatically stop the production line 1 or at least one of the production devices 38, 40, 41, 42, 43; define, by means of said decision algorithms, a corrective action to be taken to remedy said at least one current malfunctioning cause identified; signal the corrective action to be taken; indicate (for example by means of a display, not illustrated) a sequence of step-by-step instructions to guide an operator in implementing the corrective action.

The corrective action may concern one or more of the production devices 38, 40, 41, 42, 43 and/or one or more of the aforesaid accessory devices, which are involved in the current malfunctioning cause identified.

In the embodiment illustrated in Figure 1, the production line 1 comprises actuators 46 operatively associated with one or more of the production devices 38, 40, 41, 42, 43 and/or with one or more of said accessory devices. The actuators 46 are furthermore operatively connected (by means of a suitable wired and/or wireless connection) to said control unit 60.

The actuators 46 may, for example, be adapted to regulate the passage of the air at the suction devices of the retaining members 41 and may comprise shutters whose opening/closing level can be controlled automatically.

Additionally or alternatively, the actuators 46 may comprise, for example, adjustable valves and/or pressure regulators and/or temperature regulators.

The control unit is also adapted to perform a control action by sending, by the processor 62, appropriate control signals to the actuators 46 so that they implement the respective corrective action to be taken. The corrective actions are therefore automatically converted into control signals for the actuators 46 which act on the production devices 38, 40, 41, 42, 43 and/or on said accessory devices in order to remedy the malfunctioning identified, bring the anomalous operating parameters back in compliance with the respective reference values and avoid the production of defective articles.

With an appropriate feedback system, the processor 62 will be able to check that the corrective actions taken are effectively effective in bringing the anomalous operating parameters back in compliance with the respective reference values.

For example, in the event that a weight sensor (such as, for example, a load cell), as a sensor 45 associated with the first conveyor belt 48, detects an absence of material placed on the transport surface 47 at one of the feeding devices 40, the processor 62- by means of the aforesaid decision algorithms- can identify, as a malfunctioning cause currently responsible for the absence of material, a jam of material at the feeding device 40, among other possible malfunctioning causes such as, for example, a problem of material exhaustion or breakage or wear of a piece of the feeding device 40.

A similar situation can occur, for example, in the case where a weight sensor (such as, for example, a load cell), as a sensor 45 associated with the first conveyor belt 48, detects an absence of elastic flaps 24 placed on the transport surface 47 at one of the feeding devices 40. In this case, the processor 62- by means of the aforesaid decision algorithms- can identify, as a malfunctioning cause currently responsible for the absence of elastic flaps 24, a malfunctioning of the respective cutting device 43 adapted to cut a starting material to make the individual flaps 24, among other possible malfunctioning causes such as, for example, a jam of material at the respective feeding device 40 or a wrong feeding speed of the feeding device or a breakage or wear of a piece of the feeding device 40.

Consider also the situation of a glue dispensing device in one of the fixing devices 42 which is provided in its inside with a small secondary glue tank adapted to draw the glue, by means of a suitable drawing mechanism, from an external primary glue tank. In the event that a sensor 45 associated with the secondary glue tank detects that said tank is empty, the processor 62- by means of the aforesaid decision algorithms- can identify, as a malfunctioning cause currently responsible for the empty secondary tank, a problem in the glue drawing mechanism, among other possible malfunctioning causes such as, for example, an emptying of the primary glue tank.

In the aforesaid situation, also consider the case of a temperature sensor as a sensor 45 associated with the secondary glue tank of the glue dispensing device in one of the fixing devices 42. In the event that this temperature sensor detects an anomalous temperature value, the processor 62- by means of the aforesaid decision algorithms- can identify, as a malfunctioning cause currently responsible for the anomalous temperature value, a breakage or malfunctioning of the heating device (for example a coil) associated with the secondary glue tank, among other possible malfunctioning causes such as, for example, the presence of an inadequate quantity of glue in the secondary glue tank.

In addition, in the event that a frequency meter as a sensor 45 associated with an ultrasonic fixing device 42 detects an anomalous working frequency, the processor 62- by means of the aforesaid decision algorithms- can identify, as a malfunctioning cause currently responsible for the anomalous working frequency, a problem of misalignment between two sealing heads (sonotrode and anvil) of the fixing device 42, among other possible malfunctioning causes such as, for example, a problem of wear or breakage or malfunctioning or wrong setting of the sonotrode or of the fixing device 42 in general.

In each of the cases mentioned above, the processor 62 can generate an alarm signal with appropriate indications for the operator on a corrective action to be taken to remedy the current malfunctioning cause identified.

As a further example, consider also the case where a flow meter, as a sensor 45 associated with one of the fixing devices 42, detects a flow of dispensed glue that is outside respective reference values, the processor 62 can - by means of the aforesaid decision algorithms - identify as a malfunctioning cause responsible for the anomalous flow of glue, an inadequate temperature and/or humidity value within the plant that houses the production line 1, among other possible malfunctioning causes such as, for example, a breakage or malfunctioning of the heating device associated with a glue tank associated with the fixing device 42. In this case, the processor 62 may define a corrective action adapted to intervene on a temperature and/or humidity regulating device acting as actuator 46 so that it appropriately regulates the temperature and/or humidity level within the plant so as to bring the flow of glue dispensed back in compliance with the respective reference values.

A similar situation may occur, for example, in the case where a speed sensor, as a sensor 45 associated with one of the feeding devices 40, detects a material (e.g. cellulose fibre) feeding speed that is outside respective reference values. In this case, the processor 62 can identify as a malfunctioning cause, responsible for the anomalous feeding speed, an inadequate humidity value within the plant that houses the production line 1, among other possible malfunctioning causes such as, for example, a breakage or malfunctioning of the feeding device 40. Furthermore, the processor 62 can define a corrective action adapted to intervene on a humidity regulating device acting as actuator 46 so that it appropriately regulates the humidity level within the plant so as to bring the material feeding speed back in compliance with the respective reference values.

In another example, an optical sensor, as a sensor 45 associated with the transport members 38, may detect an inadequate level of cleanliness on the supporting surface 47. In this case, the processor 62 can identify as a malfunctioning cause, responsible for the inadequate level of cleanliness, an anomalous temperature value, which is outside respective reference values, at the glue tank in one of the fixing devices 42, among other possible malfunctioning causes such as, for example, a breakage or malfunctioning at one of the feeding devices 40. Furthermore, the processor 62 can define a corrective action adapted to intervene on a temperature regulating device acting as actuator 46 so that it appropriately regulates the temperature level inside the glue tank so as to bring the level of cleanliness on the supporting surface 47 back in compliance with the respective reference values.

As will be clear from the present disclosure, the advantages linked to the invention in its various aspects are manifold.

Thanks to the invention, a current malfunctioning cause of the line is identified on the basis of parameters that are directly connected to the operation of the production devices, independently of a detection of defects performed by a processing of the images acquired by the image acquisition device 70.

This advantageously allows to identify the malfunctioning causes of the line in a reliable and precise way, considering that there are malfunctionings of the line that, not affecting the quality of the articles produced or being hardly identifiable starting from a visual inspection of the articles produced by the line, cannot be identified starting from a detection of production defects performed by processing the images acquired by the image acquisition device 70.

The Applicant also observes that, by providing a control method that operates upstream of any detection of defects, the invention advantageously allows to have real-time information on malfunctioning causes that could generate production defects on the articles and, therefore, promptly signal the presence of possible defective articles and/or intervene immediately on the production line in order to prevent the production of defective articles.

In addition, the Applicant observes that there are defects (such as, for example, those related to the tightness of a gluing or of a seal or to the degree of absorbency of the absorbent padding 16) that - not being visible - are not identifiable by a processing of the images acquired by the image acquisition device 70. The latter defects, in order to be identified, would require advanced and expensive inspection devices or controls performed in the laboratory, outside the production line, on sample products.

The invention, by performing a predictive evaluation of defective articles by means of a solution that operates without *machine vision* techniques (i.e. image processing techniques), advantageously allows to predict, in the line and in an automated way, also non-visible defects, without requiring the use of advanced and expensive inspection devices or controls performed in the laboratory, outside the production line, on sample products.

Moreover, the invention advantageously allows to automatically identify, among a plurality of possible malfunctioning causes, the malfunctioning cause that is currently responsible for one or more anomalous operating parameters without requiring the intervention of experienced operators.

## Claims

1. A computer-implemented method for controlling the production of absorbent sanitary articles (2) in a production line (1), comprising the following steps:
- detecting current values of operating parameters which are indicative of the current operation of at least one production device (38, 40, 41, 42, 43) adapted to perform respective production operations on the absorbent sanitary articles (2) being processed along the production line (1);
- performing a comparison between the current values of the detected operating parameters and respective predetermined reference values;
- in the presence, among said operating parameters, of at least one anomalous operating parameter whose current value does not comply with the respective predetermined reference value, identifying, among a plurality of possible malfunctioning causes of the line (1), at least one current malfunctioning cause responsible for said at least one anomalous operating parameter;
wherein said comparison is performed independently of a detection of any defects performed on the absorbent sanitary articles (2); and
wherein said at least one current malfunctioning cause is identified starting from said at least one anomalous operating parameter by means of decision algorithms which are based on at least one of the following information: known and/or self-learned data related to correlations between said at least one anomalous operating parameter and said plurality of possible malfunctioning causes of the line; current and/or historical information manually provided by an operator in association with said at least one anomalous operating parameter and/or with said plurality of possible malfunctioning causes of the line; known and/or self-learned logic rules.

2. Control method according to claim 1, also comprising the step of acquiring article data directly correlated to the absorbent sanitary articles being processed along the production line and/or to the finished absorbent sanitary articles, in output from the line.

3. Control method according to claim 2, wherein the article data relate to at least one of: appearance, shape, weight, positioning, dimensions and contours of the articles being processed along the production line and/or to the finished absorbent sanitary articles, in output from the line.

4. Control method according to claim 1 or 2, wherein said detection of any defects is performed by processing said article data and said comparison is performed independently of said detection of any defects performed by said processing of said article data.

5. Control method according to any one of the preceding claims, also comprising the step of acquiring images of the absorbent sanitary articles being processed along the production line (1) and/or of the finished absorbent sanitary articles in output from the production line (1).

6. Control method according to any one of the preceding claims, also comprising the step of storing in a memory (61) said at least one current malfunctioning cause identified in association with said at least one anomalous operating parameter so as to enrich the self-learned data related to correlations between said at least one anomalous operating parameter and said plurality of possible malfunctioning causes of the line (1).

7. Control method according to any one of the preceding claims, also comprising the step of performing, by means of said decision algorithms, a prediction of any defects in the absorbent sanitary articles starting from said at least one current malfunctioning cause identified and based on at least one of the following information stored in said memory (61): known and/or self-learned data related to correlations between said at least one current malfunctioning cause identified and at least one known possible defect; current and/or historical information manually provided by an operator in association with said at least one current malfunctioning cause identified and/or said at least one known possible defect; known and/or self-learned logic rules.

8. Control method according to claim 8, also comprising the step of storing in a memory (61) any predicted production defects so as to enrich the self-learned data related to said correlations between said at least one current malfunctioning cause identified and said at least one known possible defect.

9. Control method according to any one of the preceding claims, also comprising the step of defining, by means of said decision algorithms, a corrective action to be taken to remedy said at least one current malfunctioning cause identified; the corrective action to be taken concerning one or more of the production devices (38, 40, 41, 42, 43) and/or one or more of accessory devices of the line (1), which are involved in the current malfunctioning cause identified.

10. Production line (1) of absorbent sanitary articles (12) comprising:
- production devices (38, 40, 41, 42, 43) configured to perform respective production operations on the absorbent sanitary articles (2) being processed along the production line (1);
- sensors (45) adapted to detect current values of operating parameters which are indicative of the current operation of at least one of the production devices (38, 40, 41, 42, 43);
- a control unit (60) comprising a memory (61) and a processor (62) configured to carry out the steps of the control method according to any of claims 1 to 12.

11. Production line (1) according to claim 10, wherein the production devices (38, 40, 41, 42, 43) comprise at least one of:
- transport members (38) adapted to support and move the absorbent sanitary articles (2) being processed along the line (1);
- feeding devices (40) adapted to feed elements of the absorbent sanitary articles (2) being processed and to place them, at least partially mutually overlapping, on a supporting surface (47) of the transport members (38);
- retaining members (41) configured to retain in position the elements placed on the supporting surface (47) of the transport members (38), preferably comprising suction devices active on retaining holes formed on the supporting surface (47) of the transport members (38);
- at least one fixing device (42) adapted to fix the elements of the absorbent sanitary articles (2) being processed together;
- at least one cutting device (43) adapted to cut elements of the absorbent sanitary articles (2) being processed and/or a continuous strip (44) of the absorbent sanitary articles (2) being processed joined together, resulting from the production process, into individual articles (2).

12. Production line (1) according to claim 10 or 11, wherein the sensors (45) comprise at least one of: temperature sensor; pressure sensor; vibration sensor; position sensor; weight sensor; quantity sensor; flow sensor; vacuum level sensor; air jet status sensor; optical sensor; tension sensor; speed sensor; torque sensor; current sensor.

13. Production line (1) according to any one of claims 10 to 12, wherein the operating parameters detected by the plurality of sensors (45) comprise at least one of: temperature; pressure; vibration; position of objects; weight; quantity; vacuum level; status of air jets; impact energy level of the cutting device (43); level of cleanliness; presence or quantity of material at a predetermined position; feeding tension of the elements of the absorbent sanitary articles (2) being processed; speed; torque; current.

14. Production lines (1) according to any one of claims 10 to 13, further comprising an image acquisition device (70) configured to acquire images of the absorbent sanitary articles being processed along the production line (1) and/or of the finished absorbent sanitary articles in output from the production line (1), wherein the processor (62) is configured to perform said detection of any defects by processing said images acquired by the image acquisition device (70).

15. Production line (1) according to any one of claims 10 to 14, wherein the processor (62) is configured to define, by means of said decision algorithms, a corrective action to be taken to remedy said at least one current malfunctioning cause identified; the corrective action to be taken concerning one or more of the production devices (38, 40, 41, 42, 43) and/or one or more of accessory devices of the line (1), which are involved in the current malfunctioning cause identified, and wherein the line comprises actuators (46) operatively associated with said control unit (60) and with at least one of the production devices (38, 40, 41, 42, 43) and/or with at least one of the accessory devices; and wherein said processor (62) is configured to automatically convert the corrective action to be taken into a control signal for the actuators (46).

## Patentansprüche

1. Computerimplementiertes Verfahren zur Steuerung der Produktion von absorbierenden Hygieneartikeln (2) in einer Produktionslinie (1), umfassend die folgenden Schritte:
- Detektieren aktueller Werte von Betriebsparametern, die indikativ für den aktuellen Betrieb mindestens einer Produktionsvorrichtung (38, 40, 41, 42, 43) sind, die angepasst ist, um jeweilige Produktionsvorgänge an den absorbierenden Hygieneartikeln (2) durchzuführen, die entlang der Produktionslinie (1) verarbeitet werden;
- Durchführen eines Vergleichs zwischen den aktuellen Werten der detektierten Betriebsparameter und jeweiligen vorbestimmten Referenzwerten;
- bei Vorhandensein mindestens eines anomalen Betriebsparameters unter den Betriebsparametern, dessen aktueller Wert nicht mit dem jeweiligen vorbestimmten Referenzwert übereinstimmt, Identifizieren, unter einer Vielzahl von möglichen Fehlfunktionsursachen der Linie (1), zumindest einer aktuellen Fehlfunktionsursache, die für den zumindest einen anomalen Betriebsparameter verantwortlich ist;
wobei der Vergleich unabhängig von einer Erkennung von Defekten durchgeführt wird, die an den absorbierenden Hygieneartikeln (2) durchgeführt wird; und
wobei die zumindest eine aktuelle Fehlfunktionsursache ausgehend von dem zumindest einen anomalen Betriebsparameter mittels Entscheidungsalgorithmen identifiziert wird, die auf zumindest einer der folgenden Informationen basieren: bekannte und/oder selbstgelernte Daten in Bezug auf Korrelationen zwischen dem zumindest einen anomalen Betriebsparameter und der Vielzahl möglicher Fehlfunktionsursachen der Linie; aktuelle und/oder historische Informationen, die manuell von einem Bediener in Assoziation mit dem zumindest einen anomalen Betriebsparameter und/oder mit der Vielzahl möglicher Fehlfunktionsursachen der Linie bereitgestellt werden; bekannte und/oder selbstgelernte Logikregeln.

2. Steuerungsverfahren nach Anspruch 1, ferner umfassend den Schritt des Erfassens von Artikeldaten, die direkt mit den absorbierenden Hygieneartikeln, die entlang der Produktionslinie verarbeitet werden und/oder mit den fertigen absorbierenden Hygieneartikeln, die aus der Produktionslinie ausgegeben werden, korreliert sind.

3. Steuerungsverfahren nach Anspruch 2, wobei sich die Artikeldaten auf mindestens eines von Folgendem beziehen: Aussehen, Form, Gewicht, Positionierung, Abmessungen und Konturen der entlang der Produktionslinie verarbeiteten Artikel und/oder der fertigen absorbierenden Hygieneartikel, die aus der Produktionslinie ausgegeben werden.

4. Steuerungsverfahren nach Anspruch 1 oder 2, wobei die Erkennung von Fehlern durch Verarbeiten der Artikeldaten durchgeführt wird und der Vergleich unabhängig von der Erkennung von Fehlern durchgeführt wird, die durch das Verarbeiten der Artikeldaten durchgeführt wird.

5. Steuerungsverfahren nach einem der vorhergehenden Ansprüche, ferner umfassend den Schritt des Erfassens von Bildern der absorbierenden Hygieneartikel, die entlang der Produktionslinie (1) verarbeitet werden, und/oder der fertigen absorbierenden Hygieneartikel, die aus der Produktionslinie (1) ausgegeben werden.

6. Steuerungsverfahren nach einem der vorhergehenden Ansprüche, ferner umfassend den Schritt des Speicherns der zumindest einen aktuellen Fehlfunktionsursache, die in Assoziation mit dem zumindest einen anomalen Betriebsparameter identifiziert wurde, in einem Speicher (61), um die selbstgelernten Daten in Bezug auf Korrelationen zwischen dem zumindest einen anomalen Betriebsparameter und der Vielzahl möglicher Fehlfunktionsursachen der Linie (1) anzureichern.

7. Steuerungsverfahren nach einem der vorhergehenden Ansprüche, ferner umfassend den Schritt des Durchführens einer Vorhersage von Defekten in den absorbierenden Hygieneartikeln mittels der Entscheidungsalgorithmen ausgehend von der zumindest einen identifizierten aktuellen Fehlfunktionsursache basierend auf mindestens einer der folgenden in dem Speicher (61) gespeicherten Informationen: bekannte und/oder selbstgelernte Daten in Bezug auf Korrelationen zwischen der zumindest einen identifizierten aktuellen Fehlfunktionsursache und zumindest einem bekannten möglichen Defekt; aktuelle und/oder historische Informationen, die manuell von einem Bediener in Assoziation mit der zumindest einen identifizierten aktuellen Fehlfunktionsursache und/oder dem zumindest einen bekannten möglichen Defekt bereitgestellt werden; bekannte und/oder selbstgelernte Logikregeln.

8. Steuerungsverfahren nach Anspruch 8, ferner umfassend den Schritt des Speicherns aller vorhergesagten Produktionsfehler in einem Speicher (61), um die selbstgelernten Daten in Bezug auf die Korrelationen zwischen der zumindest einen identifizierten aktuellen Fehlfunktionsursache und dem zumindest einen bekannten möglichen Fehler anzureichern.

9. Steuerungsverfahren nach einem der vorhergehenden Ansprüche, ferner umfassend den Schritt des Definierens einer zu ergreifenden Korrekturmaßnahme mittels der Entscheidungsalgorithmen, um die zumindest eine identifizierte aktuelle Fehlfunktionsursache zu beheben; wobei die zu ergreifende Korrekturmaßnahme eine oder mehrere der Produktionsvorrichtungen (38, 40, 41, 42, 43) und/oder eine oder mehrere der Zusatzvorrichtungen der Linie (1) betrifft, die an der identifizierten aktuellen Fehlfunktionsursache beteiligt sind.

10. Produktionslinie (1) für absorbierende Hygieneartikel (12), umfassend:
- Produktionsvorrichtungen (38, 40, 41, 42, 43), die konfiguriert sind, um jeweilige Produktionsvorgänge an den absorbierenden Hygieneartikeln (2) durchzuführen, die entlang der Produktionslinie (1) verarbeitet werden;
- Sensoren (45), die angepasst sind, um aktuelle Werte von Betriebsparametern zu detektieren, die indikativ für den aktuellen Betrieb von mindestens einer der Produktionsvorrichtungen (38, 40, 41, 42, 43) sind;
- eine Steuereinheit (60), die einen Speicher (61) und einen Prozessor (62) umfasst, die konfiguriert sind, um die Schritte des Steuerungsverfahrens nach einem der Ansprüche 1 bis 12 auszuführen.

11. Produktionslinie (1) nach Anspruch 10, wobei die Produktionsvorrichtungen (38, 40, 41, 42, 43) mindestens eines von Folgendem umfassen:
- Transportelemente (38), die angepasst sind, um die absorbierenden Hygieneartikel (2), die entlang der Linie (1) verarbeitet werden, zu tragen und zu bewegen;
- Zuführvorrichtungen (40), die angepasst sind, um Elemente der verarbeiteten absorbierenden Hygieneartikel (2) zuzuführen und sie, zumindest teilweise einander überlappend, auf einer Auflagefläche (47) der Transportelemente (38) abzulegen;
- Halteelemente (41), die konfiguriert sind, um die auf der Auflagefläche (47) der Transportelemente (38) abgelegten Elemente in Position zu halten, vorzugsweise umfassend Saugvorrichtungen, die auf Haltelöcher wirken, die auf der Auflagefläche (47) der Transportelemente (38) ausgebildet sind;
- mindestens eine Fixiervorrichtung (42), die angepasst ist, um die Elemente der verarbeiteten absorbierenden Hygieneartikel (2) zusammen zu fixieren;
- mindestens eine Schneidvorrichtung (43), die angepasst ist, um aus dem Produktionsprozess resultierende Elemente der verarbeiteten absorbierenden Hygieneartikel (2) und/oder einen zusammenhängenden Streifen (44) der verarbeiteten absorbierenden Hygieneartikel (2), die zusammengefügt sind, zu einzelnen Artikeln (2) zu schneiden.

12. Produktionslinie (1) nach Anspruch 10 oder 11, wobei die Sensoren (45) mindestens eines von Folgendem umfassen: Temperatursensor; Drucksensor; Vibrationssensor; Positionssensor; Gewichtssensor; Mengensensor; Durchflusssensor; Vakuumniveausensor; Luftstrahlstatussensor; optischer Sensor; Spannungssensor; Drehzahlsensor; Drehmomentsensor; Stromsensor.

13. Produktionslinie (1) nach einem der Ansprüche 10 bis 12, wobei die Betriebsparameter, die von der Vielzahl von Sensoren (45) detektiert werden, mindestens eines von Folgendem umfassen: Temperatur; Druck; Vibration; Position von Objekten; Gewicht; Menge; Vakuumniveau; Status von Luftstrahlen; Aufprallenergieniveau der Schneidvorrichtung (43); Sauberkeitsniveau; Vorhandensein oder Menge von Material an einer vorbestimmten Position; Zufuhrspannung der Elemente der verarbeiteten absorbierenden Hygieneartikel (2); Geschwindigkeit; Drehmoment; Strom.

14. Produktionslinien (1) nach einem der Ansprüche 10 bis 13, ferner umfassend eine Bilderfassungsvorrichtung (70), die konfiguriert ist, um Bilder der absorbierenden Hygieneartikel, die entlang der Produktionslinie (1) verarbeitet werden, und/oder der fertigen absorbierenden Hygieneartikel, die von der Produktionslinie (1) ausgegeben werden, zu erfassen, wobei der Prozessor (62) konfiguriert ist, um die Erkennung von Defekten durch Verarbeiten der von der Bilderfassungsvorrichtung (70) erfassten Bilder durchzuführen.

15. Produktionslinie (1) nach einem der Ansprüche 10 bis 14, wobei der Prozessor (62) konfiguriert ist, um mittels der Entscheidungsalgorithmen eine zu ergreifende Korrekturmaßnahme zu definieren, um die zumindest eine identifizierte aktuelle Fehlfunktionsursache zu beheben; wobei die zu ergreifende Korrekturmaßnahme eine oder mehrere der Produktionsvorrichtungen (38, 40, 41, 42, 43) und/oder eine oder mehrere der Zusatzvorrichtungen der Linie (1) betrifft, die an der identifizierten aktuellen Fehlfunktionsursache beteiligt sind, und wobei die Linie Aktoren (46) umfasst, die betriebsmäßig mit der Steuereinheit (60) und mit mindestens einer der Produktionsvorrichtungen (38, 40, 41, 42, 43) und/oder mit mindestens einer der Zusatzvorrichtungen assoziiert sind; und wobei der Prozessor (62) konfiguriert ist, um die zu ergreifende Korrekturmaßnahme automatisch in ein Steuersignal für die Aktoren (46) umzuwandeln.

## Revendications

1. Procédé mis en œuvre par ordinateur de commande de la production d'articles sanitaires absorbants (2) dans une chaîne de production (1), comprenant les étapes suivantes :
- la détection de valeurs actuelles de paramètres de fonctionnement qui sont indicatifs du fonctionnement actuel d'au moins un dispositif de production (38, 40, 41, 42, 43) adapté pour effectuer des opérations de production respectives sur les articles sanitaires absorbants (2) en cours de traitement le long de la ligne de production (1) ;
- la réalisation d'une comparaison entre les valeurs actuelles des paramètres de fonctionnement détectés et des valeurs de référence prédéterminées respectives ;
- en présence, parmi lesdits paramètres de fonctionnement, d'au moins un paramètre de fonctionnement anormal dont la valeur actuelle n'est pas conforme à la valeur de référence prédéterminée respective, l'identification, parmi une pluralité de causes de dysfonctionnement possibles de la ligne (1), d'au moins une cause de dysfonctionnement actuelle responsable dudit au moins un paramètre de fonctionnement anormal ;
dans lequel ladite comparaison est réalisée indépendamment d'une détection de tout défaut réalisée sur les articles sanitaires absorbants (2) ; et
dans lequel ladite au moins une cause actuelle de dysfonctionnement est identifiée à partir dudit au moins un paramètre de fonctionnement anormal au moyen d'algorithmes de décision qui sont basés sur au moins l'une des informations suivantes : des données connues et/ou d'auto-apprentissage relatives à des corrélations entre ledit au moins un paramètre de fonctionnement anormal et ladite pluralité de causes possibles de dysfonctionnement de la ligne ; des informations actuelles et/ou historiques fournies manuellement par un opérateur en association avec ledit au moins un paramètre de fonctionnement anormal et/ou avec ladite pluralité de causes possibles de dysfonctionnement de la ligne ; des règles logiques connues et/ou d'auto-apprentissage.

2. Procédé de commande selon la revendication 1, comprenant également l'étape d'acquisition de données d'article directement corrélées aux articles sanitaires absorbants en cours de traitement le long de la ligne de production et/ou aux articles sanitaires absorbants finis, en sortie de la ligne.

3. Procédé de commande selon la revendication 2, dans lequel les données d'article concernent au moins l'un parmi : l'apparence, la forme, le poids, le positionnement, les dimensions et les contours des articles en cours de traitement le long de la ligne de production et/ou des articles sanitaires absorbants finis, en sortie de la ligne.

4. Procédé de commande selon la revendication 1 ou 2, dans lequel ladite détection de tout défaut est réalisée par le traitement desdites données d'article et ladite comparaison est réalisée indépendamment de ladite détection de tout défaut réalisée par ledit traitement desdites données d'article.

5. Procédé de commande selon l'une quelconque des revendications précédentes, comprenant également l'étape d'acquisition d'images des articles sanitaires absorbants en cours de traitement le long de la ligne de production (1) et/ou des articles sanitaires absorbants finis en sortie de la ligne de production (1).

6. Procédé de commande selon l'une quelconque des revendications précédentes, comprenant également l'étape de stockage dans une mémoire (61) de ladite au moins une cause de dysfonctionnement actuelle identifiée en association avec ledit au moins un paramètre de fonctionnement anormal de manière à enrichir les données d'auto-apprentissage relatives à des corrélations entre ledit au moins un paramètre de fonctionnement anormal et ladite pluralité de causes de dysfonctionnement possibles de la ligne (1).

7. Procédé de commande selon l'une quelconque des revendications précédentes, comprenant également l'étape de réalisation, au moyen desdits algorithmes de décision, d'une prédiction de tout défaut dans les articles sanitaires absorbants à partir de ladite au moins une cause de dysfonctionnement actuelle identifiée et sur la base d'au moins une des informations suivantes stockées dans ladite mémoire (61) : des données connues et/ou d'auto-apprentissage relatives à des corrélations entre ladite au moins une cause de dysfonctionnement actuelle identifiée et au moins un défaut possible connu ; des informations actuelles et/ou historiques fournies manuellement par un opérateur en association avec ladite au moins une cause de dysfonctionnement actuelle identifiée et/ou ledit au moins un défaut possible connu ; des règles logiques connues et/ou d'auto-apprentissage.

8. Procédé de commande selon la revendication 8, comprenant également l'étape de stockage dans une mémoire (61) de tout défaut de production prédit afin d'enrichir les données d'auto-apprentissage relatives auxdites corrélations entre ladite au moins une cause de dysfonctionnement actuelle identifiée et ledit au moins un défaut possible connu.

9. Procédé de commande selon l'une quelconque des revendications précédentes, comprenant également l'étape de définition, au moyen desdits algorithmes de décision, d'une action corrective à entreprendre pour remédier à ladite au moins une cause de dysfonctionnement actuelle identifiée ; l'action corrective à entreprendre concernant un ou plusieurs des dispositifs de production (38, 40, 41, 42, 43) et/ou un ou plusieurs parmi des dispositifs accessoires de la ligne (1), qui sont impliqués dans la cause de dysfonctionnement actuelle identifiée.

10. Ligne de production (1) d'articles sanitaires absorbants (12) comprenant :
- des dispositifs de production (38, 40, 41, 42, 43) configurés pour réaliser des opérations de production respectives sur les articles sanitaires absorbants (2) en cours de traitement le long de la ligne de production (1) ;
- des capteurs (45) adaptés pour détecter des valeurs actuelles de paramètres de fonctionnement qui sont indicatifs du fonctionnement actuel d'au moins un des dispositifs de production (38, 40, 41, 42, 43) ;
- une unité de commande (60) comprenant une mémoire (61) et un processeur (62) configuré pour exécuter les étapes du procédé de commande selon l'une quelconque des revendications 1 à 12.

11. Ligne de production (1) selon la revendication 10, dans laquelle les dispositifs de production (38, 40, 41, 42, 43) comprennent au moins l'un parmi :
- des éléments de transport (38) adaptés pour supporter et déplacer les articles sanitaires absorbants (2) en cours de traitement le long de la ligne (1) ;
- des dispositifs d'alimentation (40) adaptés pour alimenter des éléments des articles sanitaires absorbants (2) en cours de traitement et pour les placer, au moins partiellement en chevauchement mutuel, sur une surface de support (47) des éléments de transport (38) ;
- des éléments de retenue (41) configurés pour retenir en position les éléments placés sur la surface de support (47) des éléments de transport (38), comprenant de préférence des dispositifs d'aspiration actifs sur des trous de retenue formés sur la surface de support (47) des éléments de transport (38) ;
- au moins un dispositif de fixation (42) adapté pour fixer ensemble les éléments des articles sanitaires absorbants (2) en cours de traitement ;
- au moins un dispositif de coupe (43) adapté pour couper des éléments des articles sanitaires absorbants (2) en cours de traitement et/ou une bande continue (44) des articles sanitaires absorbants (2) en cours de traitement assemblés, résultant du processus de production, en articles individuels (2).

12. Ligne de production (1) selon la revendication 10 ou 11, dans laquelle les capteurs (45) comprennent au moins l'un des éléments suivants : capteur de température ; capteur de pression ; capteur de vibration ; capteur de position ; capteur de poids ; capteur de quantité ; capteur de débit ; capteur de niveau de vide ; capteur d'état de jet d'air ; capteur optique ; capteur de tension ; capteur de vitesse ; capteur de couple ; capteur de courant.

13. Ligne de production (1) selon l'une quelconque des revendications 10 à 12, dans laquelle les paramètres de fonctionnement détectés par la pluralité de capteurs (45) comprennent au moins l'un des éléments suivants : température ; pression ; vibration ; position des objets ; poids ; quantité ; niveau de vide ; état des jets d'air ; niveau d'énergie d'impact du dispositif de coupe (43) ; niveau de propreté ; présence ou quantité de matériau à une position prédéterminée ; tension d'alimentation des éléments des articles sanitaires absorbants (2) en cours de traitement ; vitesse ; couple ; courant.

14. Ligne de production (1) selon l'une quelconque des revendications 10 à 13, comprenant en outre un dispositif d'acquisition d'images (70) configuré pour acquérir des images des articles sanitaires absorbants en cours de traitement le long de la ligne de production (1) et/ou des articles sanitaires absorbants finis en sortie de la ligne de production (1), dans laquelle le processeur (62) est configuré pour réaliser ladite détection de tout défaut en traitant lesdites images acquises par le dispositif d'acquisition d'images (70).

15. Ligne de production (1) selon l'une quelconque des revendications 10 à 14, dans laquelle le processeur (62) est configuré pour définir, au moyen desdits algorithmes de décision, une action corrective à entreprendre pour remédier à ladite au moins une cause de dysfonctionnement actuelle identifiée ; l'action corrective à entreprendre concernant un ou plusieurs des dispositifs de production (38, 40, 41, 42, 43) et/ou un ou plusieurs des dispositifs accessoires de la ligne (1), qui sont impliqués dans la cause de dysfonctionnement actuelle identifiée, et dans laquelle la ligne comprend des actionneurs (46) associés de manière opérationnelle à ladite unité de commande (60) et à au moins un des dispositifs de production (38, 40, 41, 42, 43) et/ou à au moins un des dispositifs accessoires ; et dans lequel ledit processeur (62) est configuré pour convertir automatiquement l'action corrective à entreprendre en un signal de commande pour les actionneurs (46).
